(19) Europäisches Patentamt
European Patent Office
Office européen des brevets

(11) Veröffentlichungsnummer : **0 180 889 B1**

(12) # EUROPÄISCHE PATENTSCHRIFT

(45) Veröffentlichungstag der Patentschrift : **07.06.89**

(51) Int. Cl.[4] : **C 07 D313/08**, A 61 K 31/55

(21) Anmeldenummer : **85113699.4**

(22) Anmeldetag : **28.10.85**

(54) **3-Amino-2,3-dihydro-1-benzoxepin-Verbindungen sowie Verfahren zu ihrer Herstellung und diese Verbindungen enthaltende Arzneimittel.**

(30) Priorität : **05.11.84 DE 3440296**

(43) Veröffentlichungstag der Anmeldung : **14.05.86 Patentblatt 86/20**

(45) Bekanntmachung des Hinweises auf die Patenterteilung : **07.06.89 Patentblatt 89/23**

(84) Benannte Vertragsstaaten : **AT BE CH DE FR GB IT LI LU NL SE**

(56) Entgegenhaltungen :
**EP-A- 0 024 560**
**DE-A- 1 593 760**
**CHEMICAL ABSTRACTS, Band 77, Nr. 23, 4. Dezember 1972, Seite 394, Spalten 1-2, Zusammenfassungsnr. 151867d, Columbus, Ohio, US; D. HUCKLE et al.: "4,5-Dihydro-1-benzoxepin-3(2H)-one. N-substituted 2,3-dihydro-1,5-benzoxazepin-4(5H)-ones, and related compounds"**

(73) Patentinhaber : **Kali-Chemie Pharma GmbH**
**Hans-Böckler-Allee 20 Postfach 220**
**D-3000 Hannover 1 (DE)**

(72) Erfinder : **Ohlendorf, Heinrich Wilhelm, Dr., Dipl.-Chem.**
**Schuhmachersweg 18**
**D-3008 Garbsen 2 (DE)**
Erfinder : **Ruhland, Michael, Dr., Dipl.-Biol.**
**Schwalbenflucht 24**
**D-3000 Hannover 61 (DE)**
Erfinder : **Wolf, Klaus-Ullrich, Dr. med. vet.**
**Imkersweg 6**
**D-3165 Hänigsen (DE)**

(74) Vertreter : **Lauer, Dieter, Dr.**
**c/o Kali-Chemie AG Postfach 220 Hans-Böckler-Allee 20**
**D-3000 Hannover 1 (DE)**

**EP 0 180 889 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 3-Amino-2,3-dihydro-1-benzoxepin-Verbindungen und deren Säureadditionssalze sowie diese Verbindungen enthaltende pharmazeutische Zubereitungen und Verfahren zur Herstellung dieser Verbindungen.

Aus der europäischen Patentanmeldung Nr. 24560 sind 2,3,4,5-Tetrahydro-3-amino-1-benzoxepin-5-ol-Verbindungen mit die Motilität des Magens günstig beeinflussenden Wirkungen bekannt.

Der vorliegenden Erfindung liegt die Aufgabe zugrunde, neue 3-Amino-2,3-dihydro-1-benzoxepin-Derivate mit wertvollen pharmakologischen Eigenschaften zu entwickeln.

Es wurde nun gefunden, daß die neuen 3-Amino-2,3-dihydro-1-benzoxepin-Verbindungen wertvolle pharmakologische Eigenschaften besitzen und sich insbesondere durch für Antidepressiva typische Eigenschaften auszeichnen. Aufgrund ihrer pharmakologischen Eigenschaften eignen sich die Verbindungen als Arzneimittel, insbesondere zur Behandlung von depressiven Erkrankungszuständen.

Die vorliegende Erfindung betrifft daher neue 3-Amino-2,3-dihydro-1-benzoxepin-Verbindungen der allgemeinen Formel I

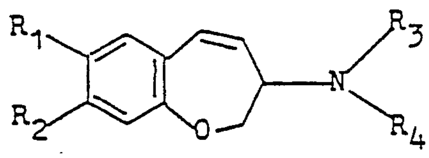

(I)

worin

$R_1$ Wasserstoff, Halogen, niederes Alkyl oder niederes Alkoxy bedeutet, und

$R_2$ Wasserstoff, Halogen, niederes Alkyl oder niederes Alkoxy bedeutet oder

einer der Substituenten $R_1$ und $R_2$ Wasserstoff ist, und der andere Nitro oder Trifluormethyl bedeutet,

$R_3$ Wasserstoff oder eine niedere Alkylgruppe bedeutet, welche gegebenenfalls an einem nicht an Stickstoff gebundenen Kohlenstoff substituiert sein kann durch Hydroxy oder niederes Alkoxy oder durch eine Phenylgruppe a

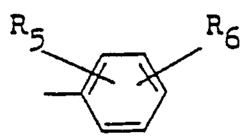

(a)

worin $R_5$ Wasserstoff, Halogen, niederes Alkyl oder niederes Alkoxy und $R_6$ Wasserstoff, Halogen, niederes Alkyl oder niederes Alkoxy bedeuten oder $R_5$ und $R_6$ an benachbarte Kohlenstoffatome gebunden sind und gemeinsam Alkylendioxy mit 1-2 Kohlenstoffatomen darstellen, oder durch eine Aminogruppe b

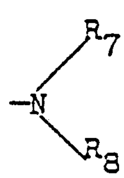

(b)

worin $R_7$ Wasserstoff oder niederes Alkyl und $R_8$ Wasserstoff oder niederes Alkyl bedeuten, oder $R_7$ und $R_8$ gemeinsam mit dem Stickstoffatom, an welches sie gebunden sind, einen 5-6-gliedrigen Heterocyclus bilden, welcher gegebenenfalls als 2. Heteroglied Sauerstoff. Schwefel oder eine $=NR_7$, Gruppe, worin $R_9$ Wasserstoff oder niederes Alkyl ist, enthält, und

$R_4$ Wasserstoff oder niederes Alkyl bedeutet, oder

$R_3$ und $R_4$ gemeinsam mit dem Stickstoffatom, an welches sie gebunden sind, einen 5-6-gliedrigen Heterocyclus bilden, welcher gegebenenfalls als 2. Heteroglied Sauerstoff, Schwefel oder eine $=NR_{10}$ Gruppe, worin $R_{10}$ Wasserstoff, niederes Alkyl oder gegebenenfalls im Phenylring durch 1-2 Substituenten aus der Gruppe Halogen, niederes Alkyl oder niederes Alkoxy substituiertes Benzyl bedeutet, enthält, und deren Säureadditionssalze.

Sofern in den Verbindungen der Formel I die Substituenten $R_1$, $R_2$, $R_3$ und $R_4$ niedere Alkylgruppen darstellen oder enthalten, können diese gerade oder verzweigt sein und vorzugsweise 1-4, insbesondere 1-2, Kohlenstoffatome enthalten.

Die Substituenten $R_1$ und $R_2$ stellen bevorzugt Wasserstoff oder auch Halogen, niederes Alkyl oder niederes Alkoxy dar. Als Halogensubstituenten $R_1$ und/oder $R_2$ kommen insbesondere Fluor, Chlor oder Brom, bevorzugt Chlor, in Frage. Sofern $R_1$ und/oder $R_2$ niederes Alkyl bedeuten, stellt dieses

insbesondere Methyl oder auch Äthyl dar. Niedere Alkoxysubstituenten stellen insbesondere Methoxy dar.

Die Substituenten $R_3$ und $R_4$ bedeuten vorzugsweise Wasserstoff oder niederes Alkyl oder bilden gemeinsam mit dem Stickstoffatom, an welches sie gebunden sind, einen 5-6-gliedrigen Heterocyclus. Insbesondere stellen $R_3$ und $R_4$ Wasserstoff oder niederes Alkyl dar. Als niedere Alkylgruppen kommen gerade oder verzweigte Alkylgruppen wie beispielsweise Methyl, Äthyl, n-Propyl, Isopropyl, n-Butyl oder verzweigte Butyle in Frage. Als günstig erweisen sich z. B. Verbindungen, worin einer der Substituenten $R_3$ und $R_4$ Wasserstoff, Methyl oder Äthyl bedeutet, und der andere eine der vorstehend genannten niederen Alkylgruppen darstellt.

Falls $R_3$ und $R_4$ gemeinsam mit dem Stickstoffatom, an welches sie gebunden sind, einen Heterocyclus bilden, welcher ein zweites Heteroglied enthält, stellt dieses insbesondere Sauerstoff oder eine Iminogruppe $= NR_{10}$ dar, worin $R_{10}$ vorzugsweise Benzyl oder niederes Alkyl bedeutet.

Als niedere Alkoxysubstituenten der Alkylgruppe $R_3$ kommen insbesondere Methoxy oder Äthoxy in Frage. Falls $R_3$ durch eine Phenylgruppe a substituiert ist, gilt für deren Substituenten $R_5$ und $R_6$ das vorstehend für die Substituenten $R_1$ und $R_2$ Gesagte. Falls $R_3$ durch eine Aminogruppe b substituiert ist, können deren Substituenten $R_7$ und $R_8$ insbesondere eine der vorstehend genannten Alkylgruppen, vorzugsweise Methyl oder Äthyl, oder auch Wasserstoff darstellen.

Besonders bevorzugt sind Verbindungen gemäss den Ansprüchen 2, 3 und 4.

Die neuen 3-Amino-2,3-dihydro-1-benzoxepin-Verbindungen der Formel I und deren Säureadditionssalze werden erfindungsgemäß erhalten, indem man aus 3-Amino-2,3,4,5-tetrahydro-1-benzoxepin-5-ol-Verbindungen der allgemeinen Formel II

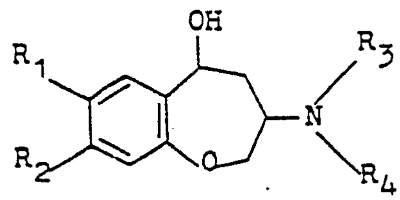

(II)

worin $R_1$, $R_2$, $R_3$ und $R_4$ obige Bedeutung besitzen, in an sich bekannten Weise Wasser abspaltet, und gegebenenfalls freie Verbindungen der Formel I in ihre Säureadditionssalze überführt oder die Säureadditionssalze in die freien Verbindungen der Formel I überführt.

Die Wasserabspaltung aus den 3-Amino-2,3,4,5-tetrahydro-1-benzoxepin-5-olen der Formel II kann nach an sich zur Dehydratisierung von Alkoholen üblichen Methoden durch Behandeln mit sauren Wasserabspaltungsmitteln erfolgen.

Beispielsweise können die Verbindungen der Formel II mit wässrigen Lösungen von unter den Reaktionsbedingungen inerten anorganischen oder organischen Säuren, gegebenenfalls unter Zusatz eines mit Wasser mischbaren inerten organischen Lösungsmittels behandelt werden. Zweckmäßigerweise werden für die Wasserabspaltung starke anorganische Säuren eingesetzt. Als Beispiele geeigneter anorganischer Säuren seien genannt Halogenwasserstoffsäuren wie Chlorwasserstoffsäure oder Bromwasserstoffsäure z. B. eine wässrige 5-32 %ige Chlorwasserstoffsäurelösung, oder auch Phosphorsäuren und Schwefelsäure. Als organische Säuren eignen sich starke organische Säuren, beispielsweise Benzolsulfonsäuren, welche im Benzolring gegebenenfalls durch niederes Alkyl oder Halogen substituiert sein können, oder niedere aliphatische Halogencarbonsäuren wie Trifluoressigsäure. Als mit Wasser mischbare Lösungsmittel, welche dem Reaktionsgemisch gegebenenfalls zugesetzt werden können, eignen sich insbesondere niedere Alkohole. Die Reaktionstemperatur und die Reaktionszeit können je nach Stärke der für die Wasserabspaltung eingesetzten Säure variieren. So können je nach Konzentration und Art der verwendeten Säure Temperaturen zwischen Raumtemperatur und Siedetemperatur des Reaktionsgemisches verwendet werden und die Reaktionszeiten können zwischen einer und mehreren Stunden betragen.

Die Verbindungen der Formel II können zur Wasserabspaltung auch mit Lewis-Säuren in einem polaren aprotischen organischen Lösungsmittel umgesetzt werden. Als Lewis-Säure wird vorzugsweise Aluminiumtrichlorid verwendet. Als Lösungsmittel eignen sich niedere Alkyläther von niederen Polyolen, z. B. niedere Glykoläther wie Äthylenglykoldimethyläther. Die Reaktion findet vorzugsweise bei erhöhter Temperatur, insbesondere Temperaturen zwischen ca. 50 °C und Siedetemperatur des Reaktionsgemisches statt.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Als pharmakologisch annehmbare Säureadditionssalze der Verbindungen der Formel I eignen sich beispielsweise deren Salze mit anorganischen Säuren, z. B. Halogenwasserstoffsäuren, insbesondere Chlorwasserstoffsäure, Schwefelsäure oder Phosphorsäuren oder mit organischen Säuren, beispielsweise niederen aliphatischen Mono- oder Dicarbonsäuren wie Milchsäure, Maleinsäure, Fumarsäure oder Essigsäure, oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure oder Cyclohexylaminosulfonsäure.

Die Verbindungen der Formel I enthalten ein chirales Kohlenstoffatom und liegen in der D- und L-Form vor. Die vorliegende Erfindung umfaßt die racemischen Gemische und die reinen optischen Isomeren der Verbindungen der Formel I.

Bei der Synthese werden je nachdem, ob von racemischen Gemischen oder optisch aktiven Verbindungen der Formel II ausgegangen wird, die Verbindungen der Formel I in Form ihrer Racemate oder als optisch aktive Verbindungen erhalten. Die optisch aktiven Verbindungen können aus den racemischen Gemischen in an sich bekannter Weise erhalten werden, z. B. durch chromatographische Trennung an chiralen Trennmaterialien oder durch Umsetzung mit geeigneten optisch aktiven Säuren, beispielsweise Weinsäure, und anschließende Auftrennung in ihre optisch aktiven Antipoden durch fraktionierte Kristallisation der gewonnenen Salze.

Die 3-Amino-2,3,4,5-tetrahydro-1-benzoxepin-5-ol-Verbindungen der Formel II sind aus der europäischen Patentanmeldung Nr. 24560 bekannt und können nach den in dieser Patentschrift beschriebenen Methoden hergestellt werden.

Die Verbindungen der Formel I und ihre pharmakologisch akzeptablen Säureadditionssalze besitzen interessante pharmakologische Eigenschaften, insbesondere für Antidepressiva typische Eigenschaften. Die neuen Verbindungen zeichnen sich durch ausgeprägte antidepressive Eigenschaften mit einem günstigen Wirkungsprofil und guter physiologischer Verträglichkeit aus. Daneben besitzen die Verbindungen auch sich vorteilhaft auf die Motilität des Magens auswirkende Eigenschaften.

Die für Antidepressiva typischen Wirkungen der Verbindungen der Formel I lassen sich in pharmakologischen Standardtests an Tieren nachweisen. So wirken die Verbindungen z. B. in der Maus antagonistisch gegenüber durch Tetrabenazin induzierter Hypothermie.

Beschreibung der pharmakologischen Untersuchungsmethoden :

1. Bestimmung der minimalen toxischen Dosis.

Männlichen Mäusen von 20-25 g Gewicht werden per os Maximaldosen von 300 mg/kg der Testsubstanz verabreicht. Die Tiere werden drei Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis angegeben.

2. Bestimmung des Tetrabenazin-Antagonismus in Mäusen.

Die antagonistische Wirkung der Substanzen auf durch Tetrabenazin induzierte Hypothermie wird in männlichen NMRI-Mäusen mit einem Körpergewicht von 18-26 g, welche vorher 16 Stunden lang nüchtern bei einer Raumtemperatur von 22 °C gehalten wurden, bestimmt.

Zur Erzeugung einer Hypothermie wird den Mäusen eine Dosis von 45 mg/kg Tetrabenazin suspendiert in einem Volumen von 10 ml/kg einer 2 %-igen Tyloselösung i. p. appliziert. 60 Min. nach der Tetrabenazinapplikation werden den Tieren die Testsubstanzen per os in einem Volumen von 10 ml/kg einer 2 %-igen Tyloselösung verabreicht. Einer Kontrolltiergruppe wird per os nur die Tyloselösung verabreicht. Die anfängliche Körpertemperatur der Tiere wird direkt vor der Tetrabenazingabe rektal mit einer Thermistor-Sonde gemessen. 1, 2 und 3 Stunden nach Applikation der Testsubstanzen werden die Körpertemperaturmessungen wiederholt. Aus den 1, 2 und 3 Stunden nach Testsubstanz- bzw. Placebogabe festgestellten Körpertemperaturwerten wird ein Mittelwert berechnet. Die Differenz zwischen diesem berechneten Mittelwert und der anfänglichen Körpertemperatur der Tiere zeigt die durch Tetrabenazin verursachte Temperaturerniedrigung an. Bei den mit antidepressiv wirksamen Testsubstanzen behandelten Tiergruppen ist die Temperatursenkung geringer als bei der nur mit Plazebo behandelten Kontrolltiergruppe. Die durch die Testsubstanzen bewirkte Verringerung der Temperatursenkung wird in % bezogen auf die bei der Kontrolltiergruppe gemessene Temperatursenkung angegeben und stellt ein Indiz für die antidepressiven Eigenschaften der Substanzen dar. Die folgende Tabelle gibt nach den vorstehend beschriebenen Testmethoden erhaltene Ergebnisse wieder. Die für die Verbindungen der Formel I angegebenen Beispielsnummern beziehen sich auf die nachstehenden Herstellungsbeispiele.

(Siehe Tabelle Seite 5 f.)

| Beispiel Nr. | minimale toxische Dosis mg/kg Maus | Antagonistische Wirkung gegenüber Tetrabenazin induzierter Hypothermie Dosis µmol/kg | % Verringerung der Hypothermie |
|---|---|---|---|
| 1 | 100 | 31,6 | 54 |
| 5 | 300 | 46.4 | 13 |
| 6 | 200 | 46.4 | 47 |
| 8 | 100 | 46.4 | 36 |
| 10 | 300 | 46.4 | 51 |
| 11 | > 300 | 46.4 | 28 |
| 12 | 300 | 46.4 | 14 |
| 14 | 100 | 46,4 | 36 |
| 16 | 200 | 46,4 | 29 |
| 17 | 100 | 46,4 | 46 |

Aufgrund der vorstehend beschriebenen Wirkungen eignen sich die Verbindungen der Formel I und ihre pharmakologisch akzeptablen Säureadditionssalze als Arzneimittel zur Behandlung und Prophylaxe von Erkrankungen und Funktionsstörungen des zentralen Nervensystems, insbesondere Erkrankungen des depressiven Formenkreises.

Die zu verwendenden Dosen variieren naturgemäß je nach Art der verwendeten Substanz, des zu behandelnden Zustandes und der Applikationsform. Z. B. werden parenterale Formulierungen im allgemeinen weniger Wirkstoff enthalten als orale Präparate. Im allgemeinen eignen sich jedoch für Applikationen an größeren Säugetieren Arzneiformen mit einem Wirkstoffgehalt und 2-30 mg pro Einzeldosis.

Als Heilmittel können die Verbindungen der Formel I und ihre physiologisch verträglichen Säureadditionssalze mit üblichen pharmazeutischen Hilfsstoffen in galenischen Zubereitungen wie z. B. Tabletten, Kapseln. Suppositorien oder Lösungen enthalten sein. Diese galenischen Zubereitungen können nach an sich bekannten Methoden hergestellt werden unter Verwendung üblicher fester Trägerstoffe, wie z. B. Milchzucker, Stärke oder Talkum oder flüssiger Verdünnungsmittel, wie z. B. Wasser, fetten Ölen oder flüssigen Paraffinen, und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Beispiel 1

3-Methylamino-2,3-dihydro-1-benzoxepin.

23,0 g 3-Methylamino-2,3,4,5-tetrahydro-1-benzoxepin-5-ol-hydrochlorid werden in 50 ml 32 %-iger wässriger Salzsäurelösung 30 min. unter Rühren auf 100 °C erhitzt. Anschließend wird die Lösung auf 100 g Eis gegossen und das Gemisch mit 70 ml 25 %-iger wässriger Ammoniaklösung alkalisch gemacht. Danach wird einmal mit 100 ml und viermal mit je 25 ml Methylenchlorid extrahiert. Die vereinigten Methylenchloridextrakte werden über Natriumsulfat getrocknet und das Lösungsmittel wird unter vermindertem Druck abdestilliert. Es werden 17,4 g 3-Methylamino-2,3-dihydro-1-benzoxepin als farbloses Öl erhalten.

Die erhaltene ölige Base wird in 25 ml Isopropanol gelöst und zur Bildung des Hydrochlorids wird gasförmiger Chlorwasserstoff in die Lösung eingeleitet. Nach dem Abkühlen wird das ausgefallene 3-Methylamino-2,3-dihydro-1-benzoxepin-hydrochlorid abfiltriert und aus Isopropanol umkristallisiert. Schmelzpunkt: 168-170 °C.

Beispiel 2

7,8-Dimethyl-3-methylamino-2,3-dihydro-1-benzoxepin.

10,0 g 7,8-Dimethyl-3-methylamino-2,3,4,5-tetrahydro-1-benzoxepin-5-ol-hydrochlorid werden in 50 ml mit Chlorwasserstoff gesättigtem Methanol gelöst und die Lösung wird 2 Stunden unter Rühren am

Rückfluß erhitzt. Anschließend wird die Lösung im Vakuum eingedampft, der Rückstand in 25 %-iger wässriger Ammoniaklösung gelöst und die ammoniakalische Lösung wie in Beispiel 1 beschrieben aufgearbeitet. Es werden 7,9 g 7,8-Dimethyl-3-methylamino-2,3-dihydro-1-benzoxepin-hydrochlorid erhalten. Schmelzpunkt : 216-218 °C.

## Beispiel 3

3-Butylamino-2,3-dihydro-1-benzoxepin.

15,0 g 3-Butylamino-2,3,4,5-tetrahydro-1-benzoxepin-5-ol-hydrochlorid werden in 80 ml 85 %-iger wässriger Phosphorsäurelösung 4 Stunden bei Raumtemperatur gerührt. Anschließend wird die Lösung auf 100 g Eis gegossen und das Gemisch wie in Beispiel 1 beschrieben aufgearbeitet. Es werden 12,1 g 3-Butylamino-2,3-dihydro-1-benzoxepin-hydrochlorid erhalten. Schmelzpunkt : 138 °C.

## Beispiel 4

3-Methylamino-2,3-dihydro-1-benzoxepin.

1,93 g 3-Methylamino-2,3,4,5-tetrahydro-1-benzoxepin-5-ol werden in 25 ml Äthylenglycoldimethyläther gegeben und unter Eiskühlung mit 1,5 g Aluminiumtrichlorid versetzt. Anschließend wird das Reaktionsgemisch 18 Stunden auf Siedetemperatur erhitzt. Danach wird das Lösungsmittel abgedampft, der Rückstand in Wasser gelöst und die Lösung mit verdünnter Natriumhydroxidlösung versetzt und mit Methylenchlorid extrahiert. Der Methylenchloridextrakt wird eingedampft und das als Rückstand verbleibende 3-Methylamino-2,3-dihydro-1-benzoxepin wird wie in Beispiel 1 beschrieben in sein Hydrochlorid überführt und dieses aus einem Methanol/Äther-Gemisch umkristallisiert. Es werden 1,9 g 3-Methylamino-2,3-dihydro-1-benzoxepin-hydrochlorid erhalten. Schmelzpunkt : 168-170 °C.

Nach den in den vorstehenden Beispielen beschriebenen Verfahren werden auch die in der nachstehenden Tabelle angegebenen 3-Amino-2,3-dihydro-1-benzoxepin-Verbindungen durch Wasserabspaltung aus entsprechenden 3-Amino-2,3,4,5-tetrahydro-1-benzoxepin-5-ol-Verbindungen hergestellt.

| Bsp. Nr. | $R_1$ /$R_2$ | | $R_3$ -N-$R_4$ | Salz | Fp. °C |
|---|---|---|---|---|---|
| 5 | H | H | H-N-$(CH_2)_2$-phen | HCl | 224-227 (Z) |
| 6 | H | H | H-N-$CH(CH_3)_2$ | HCl | 203 |
| 7 | H | H | H-N-$C_2H_5$ | HCl | 177-179 |
| 8 | H | H | pyrr- | HBr | 136-138 |
| 9 | H | H | H-N-$CH_2$-phen | HCl | 231-233 (Z) |
| 10 | H | H | $C_2H_5$-N-$C_2H_5$ | Cyc | 88-90 |
| 11 | H | H | morph- | HCl | 178-180 |
| 12 | H | H | benzpi- | 2 HCl | 258-260 |
| 13 | H | H | pipe- | HBr | 160-162 |
| 14 | 7-Cl | H | H-N-$CH_3$ | HCl | 208-209 |
| 15 | 7-Cl | 8-$CH_3$ | H-N-$CH_3$ | HCl | 243-254 |
| 16 | 7-$C_2H_5$ | H | H-N-$CH_3$ | HCl | 238-240 (Z) |
| 17 | H | H | $CH_3$-N-$C_2H_5$ | HBr | 126-128 |

(Fortsetzung)

| Bsp. Nr. | $R_1$ /$R_2$ | | $R_3$ -N-$R_4$ | Salz | Fp. °C |
|---|---|---|---|---|---|
| 18 | H | H | H-N-H | HCl | 220-223 (Z) |
| 19 | H | H | $CH_3$ -N-$CH_3$ | Mal | 135-137 |
| 20 | 8-$CF_3$ | H | H-N-$CH_3$ | Ba | öl |
| 21 | 7-$NO_2$ | H | H-N-$CH_3$ | Ba | öl |
| 22 | 7-Cl | 8-Cl | H-N-$CH_3$ | HCl | 234-235 (Z) |
| 23 | 8-Cl | H | H-N-$CH_3$ | HCl | 210-211 (Z) |
| 24 | 7-Br | H | H-N-$CH_3$ | HCl | 258-260 (Z) |
| 25 | 7-$OCH_3$ | H | H-N-$CH_3$ | Ba | öl |
| 26 | 8-$OCH_3$ | H | H-N-$CH_3$ | Ba | öl |
| 27 | H | H | H-N-$(CH_2)_2$-$OCH_3$ | HCl | 88 - 90 |
| 28 | H | H | H-N-$(CH_2)_2$-OH | Mal | 98 - 99 |
| 29 | H | H | $CH_3$ -pi- | 2 HCl | 225 - 227 (Z) |
| 30 | H | H | $CH_3$ -N-$CH_2$ -(2-Cl-phen) | HCl | 180 - 181 |
| 31 | H | H | $CH_3$ -N-$CH_2$ -(3-$CH_3$O-phen) | HCl | 176 - 177 |
| 32 | H | H | H-N-$CH_2$-(4-$CH_3$ -phen) | Mal | 125 - 127 |
| 33 | H | H | H-N-$(CH_2)_3$-N$(CH_3)_2$ | 2 HCl | 199 - 201 |
| 34 | H | H | $CH_3$ -N-$CH_2$ -(2,4-di-Cl-phen) | HCl | 144 - 146 |

HCl = Hydrochlorid,
Mal = Maleinat,
Cyc = Cyclohexylaminosulfonat,
pyrr = Pyrrolidino,
benzpi = 4-Benzylpiperazin,
$CH_3$-pi = 4-Methylpiperazin,
Z = unter Zersetzung
HBr = Hydrobromid,
Ba = Base,
phen = Phenyl,
morph = Morpholin,
pipe = Piperidin,
Öl = ölig,

Beispiel I

3-Methylamino-2,3-dihydro-1-benzoxepin-hydrochlorid enthaltende Tabletten.

Es werden Tabletten in folgender Zusammensetzung pro Tablette hergestellt :

| | |
|---|---|
| 3-Methylamino-2,3-dihydro-1-benzoxepin-hydrochlorid | 20 mg |
| Maisstärke | 60 mg |
| Milchzucker | 135 mg |
| Gelatine (als 10 %-ige Lösung) | 6 mg |

Der Wirkstoff, die Maisstärke und der Milchzucker werden mit der 10 %-igen Gelatine-Lösung eingedickt. Die Paste wird zerkleinert und das entstandene Granulat wird auf ein geeignetes Blech gebracht und getrocknet. Das getrocknete Granulat wird durch eine Zerkleinerungsmaschine geleitet und in einem Mixer mit weiteren folgenden Hilfsstoffen vermischt :

| | |
|---|---|
| Talkum | 5 mg |
| Magnesiumstearat | 5 mg |
| Maisstärke | 9 mg |

und sodann zu Tabletten von 240 mg verpreßt.

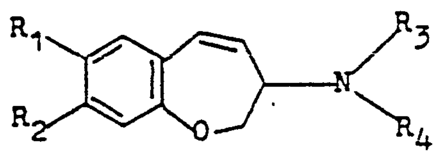

(I)

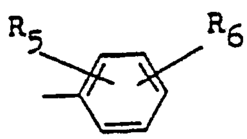

(a)

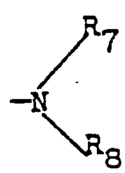

(b)

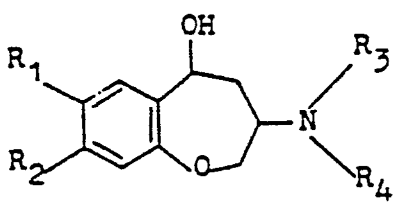

(II)

**Patentansprüche**

1. 3-Amino-2,3-dihydro-1-benzoxepin-Verbindungen der allgemeinen Formel I

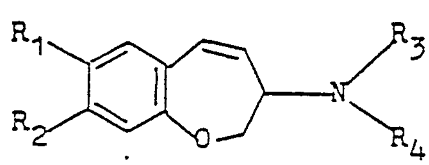

(I)

worin

$R_1$ Wasserstoff, Halogen, niederes Alkyl oder niederes Alkoxy bedeutet, und

$R_2$ Wasserstoff, Halogen, niederes Alkyl oder niederes Alkoxy bedeutet, oder

einer der Substituenten $R_1$ und $R_2$ Wasserstoff ist und der andere Nitro oder Trifluormethyl bedeutet,

$R_3$ Wasserstoff oder eine niedere Alkylgruppe bedeutet, welche gegebenenfalls an einem nicht an Stickstoff gebundenen Kohlenstoffatom substituiert sein kann durch Hydroxy oder niederes Alkoxy oder durch eine Phenylgruppe a

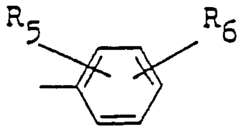

(a)

worin $R_5$ Wasserstoff, Halogen, niederes Alkyl oder niederes Alkoxy und $R_6$ Wasserstoff, Halogen, niederes Alkyl oder niederes Alkoxy bedeuten oder $R_5$ und $R_6$ an benachbarte Kohlenstoffatome gebunden sind und gemeinsam Alkylendioxy mit 1-2 Kohlenstoffatomen darstellen, oder durch eine Aminogruppe b

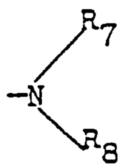

(b)

worin $R_7$ Wasserstoff oder niederes Alkyl und $R_8$ Wasserstoff oder niederes Alkyl bedeuten, oder $R_7$ und $R_8$ gemeinsam mit dem Stickstoffatom, an welches sie gebunden sind, einen 5-6-gliedrigen Heterocyclus bilden, welcher gegebenenfalls als 2. Heteroglied Sauerstoff, Schwefel oder eine $=NR_9$ Gruppe, worin $R_9$ Wasserstoff oder niederes Alkyl ist, enthält, und

$R_4$ Wasserstoff oder niederes Alkyl bedeutet, oder

$R_3$ und $R_4$ gemeinsam mit dem Stickstoffatom, an welches sie gebunden sind, einen 5-6-gliedrigen Heterocyclus bilden, welcher gegebenenfalls als 2. Heteroglied Sauerstoff, Schwefel oder eine $=NR_{10}$ Gruppe, worin $R_{10}$ Wasserstoff, niederes Alkyl oder gegebenenfalls im Phenylring durch 1-2 Substituenten aus der Gruppe Halogen, niederes Alkyl oder niederes Alkoxy substituiertes Benzyl bedeutet, enthält, und deren Säureadditionssalze.

2. 3-Amino-2,3-dihydro-1-benzoxepin-Verbindungen gemäß Anspruch 1, worin $R_1$ Wasserstoff, Halogen, niederes Alkyl oder niederes Alkoxy bedeutet, $R_2$ Wasserstoff, Halogen, niederes Alkyl oder niederes Alkoxy bedeutet, $R_3$ Wasserstoff oder eine niedere Alkylgruppe und $R_4$ Wasserstoff oder eine niedere Alkylgruppe bedeuten.

3. 3-Amino-2,3-dihydro-1-benzoxepin-Verbindungen gemäß Anspruch 2, worin $R_1$ Wasserstoff, Chlor, Brom oder niederes Alkyl mit 1-2 Kohlenstoffatomen bedeutet, $R_2$ Wasserstoff, Chlor, Brom oder niederes Alkyl mit 1-2 Kohlenstoffatomen bedeutet und $R_3$ und $R_4$ die in Anspruch 2 angegebene Bedeutung besitzen.

4. 3-Methylamino-2,3-dihydro-1-benzoxepin und dessen Säureadditionssalze.

5. Arzneimittel enthaltend eine pharmakologisch wirksame Menge einer Verbindung der Formel I gemäß Anspruch 1 und übliche pharmazeutische Hilfs- und/oder Trägerstoffe.

6. Verfahren zur Herstellung von 3-Amino-2,3-dihydro-1-benzoxepin-Verbindungen der allgemeinen Formel I

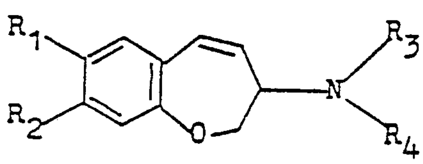

(I)

worin

$R_1$ Wasserstoff, Halogen, niederes Alkyl oder niederes Alkoxy bedeutet, und

$R_2$ Wasserstoff, Halogen, niederes Alkyl oder niederes Alkoxy bedeutet, oder

einer der Substituenten $R_1$ und $R_2$ Wasserstoff ist, und der andere Nitro oder Trifluormethyl bedeutet,

$R_3$ Wasserstoff oder eine niedere Alkylgruppe bedeutet, welche gegebenenfalls an einem nicht an Stickstoff gebundenen Kohlenstoffatom substituiert sein kann durch Hydroxy oder niederes Alkoxy oder durch eine Phenylgruppe a

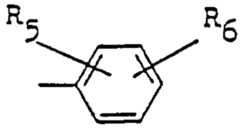

(a)

worin $R_5$ Wasserstoff, Halogen, niederes Alkyl oder niederes Alkoxy und $R_6$ Wasserstoff, Halogen, niederes Alkyl oder niederes Alkoxy bedeuten oder $R_5$ und $R_6$ an benachbarte Kohlenstoffatome gebunden sind und gemeinsam Alkylendioxy mit 1-2 Kohlenstoffatomen darstellen, oder durch eine Aminogruppe b

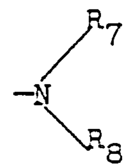

(b)

worin $R_7$ Wasserstoff oder niederes Alkyl und $R_8$ Wasserstoff oder niederes Alkyl bedeuten, oder $R_7$ und $R_8$ gemeinsam mit dem Stickstoffatom, an welches sie gebunden sind, einen 5-6-gliedrigen Heterocyclus bilden, welcher gegebenenfalls als 2. Heteroglied Sauerstoff, Schwefel oder eine $=NR_9$ Gruppe, worin $R_9$ Wasserstoff oder niederes Alkyl ist, enthält, und

$R_4$ Wasserstoff oder niederes Alkyl bedeutet, oder

$R_3$ und $R_4$ gemeinsam mit dem Sauerstoffatom, an welches sie gebunden sind, einen 5-6-gliedrigen Heterocyclus bilden, welcher gegebenenfalls als 2. Heteroglied Sauerstoff, Schwefel oder eine $=NR_{10}$ Gruppe, worin $R_{10}$ Wasserstoff, niederes Alkyl oder gegebenenfalls im Phenylring durch 1-2 Substituenten aus der Gruppe Halogen, niederes Alkyl oder niederes Alkoxy substituiertes Benzyl bedeutet, enthält, und deren Säureadditionssalzen dadurch gekennzeichnet, daß man aus Verbindungen der allgemeinen Formel II

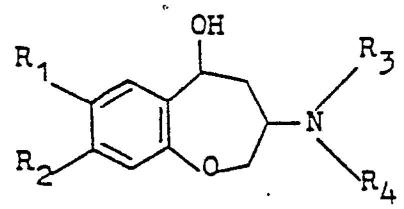

(II)

worin $R_1$, $R_2$, $R_3$ und $R_4$ obige Bedeutung besitzen, Wasser abspaltet, und gegebenenfalls freie Verbindungen der Formel I in ihre Säureadditionssalze überführt oder die Säureadditionssalze in die freien Verbindungen der Formel I überführt.

**Claims**

1. 3-amino-2,3-dihydro-1-benzoxepine compounds of the general Formula I,

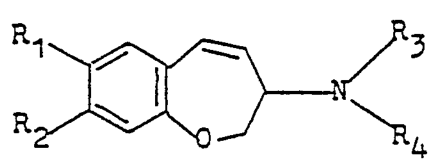

(I)

wherein

$R_1$ is hydrogen, halogen, lower alkyl or lower alkoxy, and

$R_2$ is hydrogen, halogen, lower alkyl or lower alkoxy, or

one of the substituents $R_1$ and $R_2$ is hydrogen and the other is nitro or trifluoromethyl,

$R_3$ is hydrogen or a lower alkyl group, which may optionally be substituted on a carbon atom which is not bonded to nitrogen by hydroxy or lower alkoxy or by a phenyl group a,

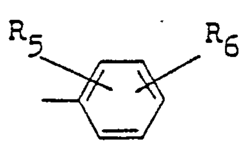

(a)

wherein $R_5$ is hydrogen, halogen, lower alkyl or lower alkoxy, and $R_6$ is hydrogen, halogen, lower alkyl or lower alkoxy, or $R_5$ and $R_6$ are bonded to adjacent carbon atoms and together represent alkylene dioxy with 1-2 carbon atoms, or by an amino group b,

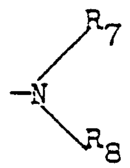

(b)

wherein $R_7$ is hydrogen or lower alkyl and $R_8$ is hydrogen or lower alkyl, or $R_7$ and $R_8$ together with the nitrogen atom to which they are bonded form a 5-6-member heterocycle which optionally contains as a second hetero member oxygen, sulphur or an $=NR_9$ group, wherein $R_9$ is hydrogen or lower alkyl, and

$R_4$ is hydrogen or lower alkyl, or

$R_3$ and $R_4$ together with the nitrogen atom to which they are bonded form a 5-6-member heterocycle which optionally contains as a second hetero member oxygen, sulphur or an $=NR_{10}$ group, wherein $R_{10}$ is hydrogen, lower alkyl or benzyl optionally substituted in the phenyl ring by 1-2 substituents from the group of halogen, lower alkyl or lower alkoxy,

and their acid addition salts.

2. 3-amino-2,3-dihydro-1-benzoxepine compounds according to Claim 1, wherein $R_1$ is hydrogen, halogen, lower alkyl or lower alkoxy, $R_2$ is hydrogen, halogen, lower alkyl or lower alkoxy, $R_3$ is hydrogen or a lower alkyl group and $R_4$ is hydrogen or a lower alkyl group.

3. 3-amino-2,3-dihydro-1-benzoxepine compounds according to Claim 2, wherein $R_1$ is hydrogen, chlorine, bromine or lower alkyl with 1-2 carbon atoms, $R_2$ is hydrogen, chlorine, bromine or lower alkyl with 1-2 carbon atoms and $R_3$ and $R_4$ have the meanings given in Claim 2.

4. 3-methylamino-2,3-dihydro-1-benzoxepine and its acid addition salts.

5. Medicament containing a pharmacologically effective quantity of a compound of Formula I in accordance with Claim 1 and conventional pharmaceutical auxiliaries and/or carriers.

6. Method for preparing 3-amino-2,3-dihydro-1-benzoxepine compounds of general Formula I,

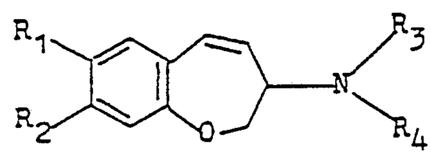

(I)

wherein

$R_1$ is hydrogen, halogen, lower alkyl or lower alkoxy, and

$R_2$ is hydrogen, halogen, lower alkyl or lower alkoxy, or

one of the substituents $R_1$ and $R_2$ is hydrogen and the other is nitro or trifluoromethyl,

$R_3$ is hydrogen or a lower alkyl group, which may optionally be substituted on a carbon atom which is not bonded to nitrogen by hydroxy or lower alkoxy or by a phenyl group a,

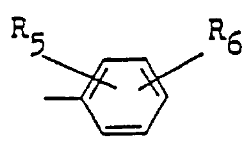

(a)

wherein $R_5$ is hydrogen, halogen, lower alkyl or lower alkoxy, and $R_6$ is hydrogen, halogen, lower alkyl or lower alkoxy, or $R_5$ and $R_6$ are bonded to adjacent carbon atoms and together represent alkylene dioxy with 1-2 carbon atoms, or by an amino group b,

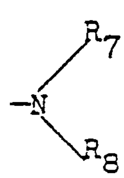

(b)

wherein $R_7$ is hydrogen or lower alkyl and $R_8$ is hydrogen or lower alkyl, or $R_7$ and $R_8$ together with the nitrogen atom to which they are bonded form a 5-6-member heterocycle which optionally contains as a second hetero member oxygen, sulphur or an $=NR_9$ group, wherein $R_9$ is hydrogen or lower alkyl, and

$R_4$ is hydrogen or lower alkyl, or

$R_3$ and $R_4$ together with the oxygen atom to which they are bonded form a 5-6-member heterocycle which optionally contains as a second hetero member oxygen, sulphur or an $=NR_{10}$ group, wherein $R_{10}$ is hydrogen, lower alkyl or benzyl optionally substituted in the phenyl ring by 1-2 substituents from the group of halogen, lower alkyl or lower alkoxy,

and their acid addition salts, characterised in that water is split off from compounds of general Formula II,

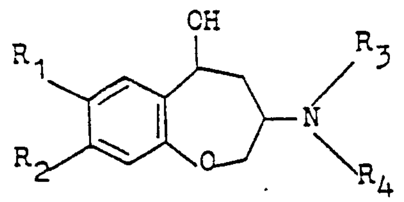

(II)

wherein $R_1$, $R_2$, $R_3$ and $R_4$ have the above meanings, and optionally free compounds of Formula I are converted into their acid addition salts or the acid addition salts are converted into the free compounds of Formula I.

**Revendications**

1. Composés de 3-amino-2,3-dihydro-1-benzoxépine de formule générale I

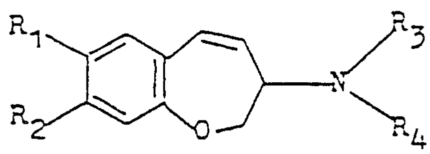

(I)

dans laquelle

$R_1$ représente l'hydrogène, un halogène, un alkyle inférieur ou un alcoxy inférieur et

$R_2$ représente l'hydrogène, un halogène, un alkyle inférieur ou un alcoxy inférieur, ou

un des substituants $R_1$ et $R_2$ est l'hydrogène et l'autre représente un nitro ou un trifluorométhyle,

$R_3$ représente l'hydrogène ou un groupe alkyle inférieur qui peut être éventuellement substitué sur un atome de carbone non lié à l'azote par un hydroxy, un alcoxy inférieur ou par un groupe phényle a

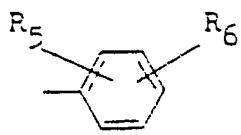

(a)

où $R_5$ représente l'hydrogène, un halogène, un alkyle inférieur ou un alcoxy inférieur et $R_6$ représente l'hydrogène, un halogène, un alkyle inférieur ou un alcoxy inférieur où $R_5$ et $R_6$ sont liés à des atomes de carbone voisins et constituent ensemble un alkylènedioxy avec 1 à 2 atomes de carbone ou par un groupe amino b

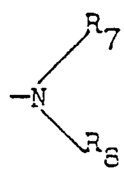

(b)

où $R_7$ représente l'hydrogène ou un alkyle inférieur et $R_8$ représente l'hydrogène ou un alkyle inférieur où $R_7$ et $R_8$ forment, ensemble avec l'atome d'azote auquel ils sont liés, un hétérocycle penta- à hexagonal qui contient éventuellement comme 2ème hétéro-atome de l'oxygène, du soufre ou un groupe $=NR_9$ où $R_9$ représente l'hydrogène ou un alkyle inférieur et

$R_4$ représente l'hydrogène ou un alkyle inférieur ou

$R_3$ et $R_4$ forment, ensemble avec l'atome d'azote auquel ils sont liés, un hétérocycle penta- à hexagonal qui contient éventuellement comme 2ème hétéro-atome de l'oxygène, du soufre ou un groupe $=NR_{10}$ où $R_{10}$ représente l'hydrogène, un alkyle inférieur ou un benzyle éventuellement substitué dans le noyau phényle par 1-2 substituants du groupe des halogènes, des alkyles inférieurs ou des alcoxys inférieurs et leurs sels d'addition avec des acides.

2. Composés de 3-amino-2,3-dihydro-1-benzoxépine selon la revendication 1, dans lesquels $R_1$ représente l'hydrogène, un halogène, un alkyle inférieur ou un alcoxy inférieur, $R_2$ représente l'hydrogène, un halogène, un alkyle inférieur ou un alcoxy inférieur, $R_3$ représente l'hydrogène ou un groupe alkyle inférieur et $R_4$ représente l'hydrogène ou un groupe alkyle inférieur.

3. Composés de 3-amino-2,3-dihydro-1-benzoxépine selon la revendication 2, dans lesquels $R_1$

représente l'hydrogène, le chlore, le brome ou un alkyle inférieur avec 1-2 atomes de carbone, $R_2$ représente l'hydrogène, le chlore, le brome ou un alkyle inférieur avec 1-2 atomes de carbone et $R_3$ et $R_4$ ont la signification indiquée dans la revendication 2.

4. 3-méthylamino-2,3-dihydro-1-benzoxépine et ses sels d'addition avec des acides.

5. Médicament contenant une quantité pharmacologiquement efficace d'un composé de formule générale I selon la revendication 1 et les adjuvants et/ou excipients pharmaceutiques usuels.

6. Procédé de préparation de composés de 3-amino-2,3-dihydro-1-benzoxépine de formule générale I

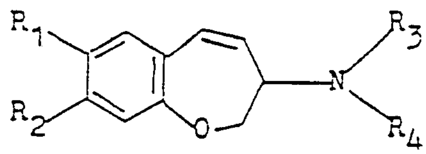

(I)

dans laquelle

$R_1$ représente l'hydrogène, un halogène, un alkyle inférieur ou un alcoxy inférieur et

$R_2$ représente l'hydrogène, un halogène, un alkyle inférieur ou un alcoxy inférieur, ou un des substituants $R_1$ et $R_2$ est l'hydrogène et l'autre représente un nitro ou un trifluorométhyle,

$R_3$ représente l'hydrogène ou un groupe alkyle inférieur qui peut être éventuellement substitué sur un atome de carbone non lié à l'azote par un hydroxy, un alcoxy inférieur ou par un groupe phényle a

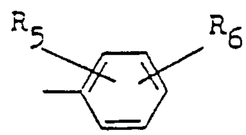

(a)

où $R_5$ représente l'hydrogène, un halogène, un alkyle inférieur ou un alcoxy inférieur et $R_6$ représente l'hydrogène, un halogène, un alkyle inférieur ou un alcoxy inférieur où $R_5$ et $R_6$ sont liés à des atomes de carbone voisins et constituent ensemble un alkylènedioxy avec 1 à 2 atomes de carbone ou par un groupe amino b

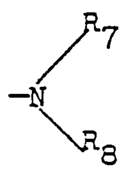

(b)

où $R_7$ représente l'hydrogène ou un alkyle inférieur et $R_8$ représente l'hydrogène ou un alkyle inférieur où $R_7$ et $R_8$ forment, ensemble avec l'atome d'azote auquel ils sont liés, un hétérocycle penta- à hexagonal qui contient éventuellement comme 2ème hétéro-atome de l'oxygène, du soufre ou un groupe $=NR_9$ où $R_9$ représente l'hydrogène ou un alkyle inférieur et

$R_4$ représente l'hydrogène ou un alkyle inférieur ou

$R_3$ et $R_4$ forment, ensemble avec l'atome d'azote auquel ils sont liés, un hétérocycle penta-hexagonal qui contient éventuellement comme 2ème hétéro-atome de l'oxygène, du soufre ou un groupe $=NR_{10}$ où $R_{10}$ représente l'hydrogène, un alkyle inférieur ou, un benzyle éventuellement substitué dans le noyau phényle par 1-2 substituants du groupe des halogènes, d'un alkyle inférieur ou d'un alcoxy inférieur et leurs sels d'addition avec des acides,

caractérisé en ce qu'on élimine de l'eau à partir des composés de formule générale II

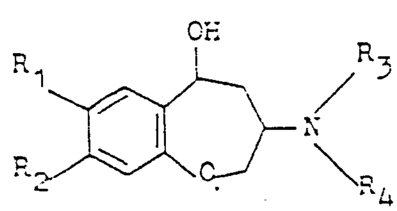

(II)

où $R_1$, $R_2$, $R_3$ et $R_4$ ont la signification précédente et qu'on transforme, le cas échéant, des composés libres de formule I en leurs sels d'addition avec des acides ou qu'on transforme les sels d'addition avec des acides en composés libres de formule I.